# EUROPEAN PATENT APPLICATION

(11) **EP 4 588 495 A1**
(43) Date of publication of application: **23.07.2025**
(21) Application number: 23865285.3
(22) Date of filing: 30.08.2023
(51) Int. Cl.: A61M 5/145, A61M 5/20

(54) **INJECTOR, AND METHOD FOR INJECTING SOLUTION USING SAID INJECTOR**

(30) Priority: 13.09.2022 JP 2022145330
(71) Applicant: Daicel Corporation, Osaka-shi, Osaka 530-0011 (JP)
(72) Inventor: TERAI, Kazuhiro, Tokyo 108-8230 (JP); YAMAMOTO, Yuzo, Tokyo 108-8230 (JP); IGA, Hiromitsu, Tokyo 108-8230 (JP)
(74) Representative: Grünecker Patent- und Rechtsanwälte PartG mbB
(86) International application number: PCT/JP2023/031451
(87) International publication number: WO 2024/057929

(57) **Abstract**

At least the following is provided. That is, provided is a technique by which it is possible to achieve high physiological activity when a solution containing a biofunctional substance exhibiting physiological activity in an injection target is injected into the injection target. An injector for injecting a solution containing a biofunctional substance into an injection target includes an accommodation part configured to accommodate the solution containing the biofunctional substance, a pressurization part configured to pressurize the solution containing the biofunctional substance during actuation, and an ejection part insertable into the injection target and including an inlet port configured to facilitate inflow of the solution containing the biofunctional substance from the accommodation part when the solution containing the biofunctional substance is pressurized by the pressurization part, and an outlet port configured to facilitate ejection of the flowing solution containing the biofunctional substance into the injection target. An ejection rate of the solution containing the biofunctional substance exceeds 83.3 µL/s.

## Description

### Technical Field

The present disclosure relates to an injector and a method for injecting a solution using the injector.

### Background Art

Examples of an injector that injects a medical liquid into an injection target include a needle-equipped injector that performs injection through an injection needle and a needleless injector that performs injection without using an injection needle. Further, a catheter including an injection needle and a drive source, a multi-hole injection needle, and the like are also utilized to transport a medical liquid to an injection target.

With regard to the needle-equipped injector, inserting an injection needle into the injection target, pushing a plunger and then injecting the medical liquid, usually slowly, disperses the medical liquid into the injection target, allowing a medicinal effect to be appropriately exhibited. In particular, in a case in which the medical liquid is administered without depending on blood flow, a very small amount of the medical liquid is slowly administered (for example, at about 0.0033 µL/s). Further, in many cases, the medical liquid is "slowly" administered on the basis of the subjectivity of the administrator.

In contrast, a needleless injector is used to administer a medical liquid into a living body from a skin surface by instantly pushing out the medical liquid from a nozzle tip using compressed gas, a spring force, or the like as a driving force (Non-Patent Document 1). Further, a needleless injector that utilizes combustion energy of an ignition agent as a driving force has been developed (Patent Document 1).

The needleless injector that utilizes the combustion energy of an ignition agent as ejection energy is capable of delivering a medical liquid to the nucleus or cytosol of a cell by instantly pushing out and administering the medical liquid. The needleless injector can deliver active ingredients to the injection target without necessarily requiring viral vectors or lipid carriers, and thus the usefulness of the needleless injector has been attracting attention also from the viewpoint of a drug delivery system (DDS), and application of the needleless injector to delivery of various medical liquids using a low-molecular-weight molecule, a peptide, a protein, an antibody, or the like having an anticancer action has been studied. In particular, in the fields of vaccines and immunization, it has been reported that the needleless injector enables an increase in antibody titer or the induction of cellular immunity (Non-Patent Document 2). There is also a report that intradermal administration of plasmid deoxyribonucleic acid (DNA) to an experimental animal using a needleless injector significantly enhances gene expression as compared with administration using a classical needle-equipped injector (Non-Patent Document 3).

A medical liquid administered from the skin surface using a needleless injector is delivered intradermally through the stratum corneum of the skin as a jet stream (Non-Patent Document 1). By adjusting the output, it is possible to deliver a medical liquid subcutaneously or intramuscularly (Non-Patent Document 4).

### Citation List

### Patent Document

Patent Document 1: JP 2012-61269 A

### Non-Patent Document

Non-Patent Document 1: Clin. Cosmet. Investig. Dermatol., 2018 May 1, 11: 231 to 238
Non-Patent Document 2: AAPS PharmSciTech., 2019 Dec 9, 21(1): 19
Non-Patent Document 3: Journal of Pharmaceutical Sciences, 2019, 108: 2415 to 2420.
Non-Patent Document 4: Gene, 2021, 788: 145664

### Summary of Invention

### Technical Problem

In a case in which the inside of a living body is the injection target, a classical needle-equipped injector is typically used as the injector for injecting a medical liquid, but there is room for improvement in enhancement of medical efficacy.

An object of the present disclosure is at least the following. That is, provided is a technique by which it is possible to achieve, in a case in which the inside of a living body is an injection target, high physiological activity when a solution containing a biofunctional substance exhibiting physiological activity in an injection target is injected into the injection target.

### Solution to Problem

As a result of intensive studies to solve the above problems, the present inventors have found that the above problems can be solved by an injector including an ejection part insertable into an injection target and capable of ejecting a solution at a predetermined high ejection rate.

That is, an overview of the present disclosure is as described below.
[1] An injector for injecting a solution containing a biofunctional substance into an injection target, the injector including:
   an accommodation part configured to accommodate the solution containing the biofunctional substance;
   a pressurization part configured to pressurize the solution containing the biofunctional substance during actuation; and
   an ejection part insertable into the injection target and including
   an inlet port configured to facilitate inflow of the solution containing the biofunctional substance from the accommodation part when the solution containing the biofunctional substance is pressurized by the pressurization part, and
   an outlet port configured to facilitate ejection of the flowing solution containing the biofunctional substance into the injection target,
   in which
   an ejection rate of the solution containing the biofunctional substance exceeds 83.3 µL/s.
[2] The injector according to [1], in which the ejection rate of the solution containing the biofunctional substance is 200 µL/s or higher.
[3] The injector according to [1] or [2], in which an inner diameter of the ejection part is 0.41 mm or less.
[4] The injector according to any one of [1] to [3], in which an outer diameter of the ejection part is 0.72 mm or less.
[5] The injector according to any one of [1] to [4], in which the ejection part is an injection needle.
[6] The injector according to any one of [1] to [5], in which the biofunctional substance is one or more selected from the group consisting of a nucleic acid, a peptide, a protein, and a low-molecular-weight compound.
[7] The injector according to any one of [1] to [6], in which the injection target is one or more selected from the group consisting of an articular cavity, a spleen, an eyeball, skin, a muscle, a bone, cartilage, marrow, a ligament, a brain, a spinal cord, a lung, a liver, a heart, a kidney, a pancreas, a gall bladder, a digestive tract, a bladder, a reproductive organ, a lymph node, a lymphatic network, a blood vessel, a tumor arising in any one thereof, and mucosa and connective tissue associated with any one thereof.
[8] A method including injecting a solution containing a biofunctional substance into an injection target, using the injector described in any one of [1] to [7].

### Advantageous Effects of Invention

The present disclosure can exhibit at least the following effect. That is, there can be exhibited an effect of providing a technique by which it is possible to achieve, in a case in which the inside of a living body is an injection target, high physiological activity when a solution containing a biofunctional substance exhibiting physiological activity in an injection target is injected into the injection target.

### Brief Description of Drawings

FIG. 1 is an overall view of an injector according to an aspect of the present disclosure.
FIG. 2 is a cross-sectional view illustrating the vicinity of a tip end portion of the injector illustrated in FIG. 1.
FIG. 3 is an overall view of an injector according to a modified example.
FIG. 4 is a cross-sectional view of the injector illustrated in FIG. 3.
FIG. 5 is a graph showing Luc activity (total RFU) in a joint for a 30G needle-equipped 2.5-mL Hamilton syringe (Syr) group (n = 4), a 30G-pNFI-25/40 (25/40 device) group (n = 4), and a 30G-pNFI-35/40 (35/40 device) group (n = 4). Mean + SD.
FIG. 6 is a graph showing Luc activity (total RFU) in a joint for a 30G needle-equipped 25-mL Hamilton syringe - administration rate 0.25 µL/s (administration rate 0.25 µL/s) group (n = 4), a 30G needle-equipped 25-mL Hamilton syringe - administration rate 83.3 µL/s (administration rate 83.3 µL/s) group (n = 8), and a 30G-pNFI-35/40 group (n = 8). Mean + SD, Student t-test, *P < 0.05.
FIG. 7 is graph showing Luc activity (total RFU) in a joint for a 30G-pNFI-35/40 (35/40) group, a 30G-pNFI-55/40 (55/40) group, and a 30G-pNFI-75/40 (75/40) group. Mean + SD.
FIG. 8 is a graph showing ejection rates of a solution when using the 30G-pNFI-25/40, the 30G-pNFI-35/40, the 30G-pNFI-55/40, the 30G-pNFI-75/40, and pNFI-35/40.
FIG. 9 is a graph showing Luc activity (total RFU) in a spleen in a case of administration using the 30G-pNFI-35/40 and a 30G needle-equipped insulin syringe (30G-Syr). Mean + SD.
FIG. 10 is a graph showing Luc activity (total RFU) in a spleen in a case of administration using the 30G-pNFI-35/40 and a 30G needle-equipped Hamilton syringe (30G-Syr/pump). Mean + SD.
FIG. 11 is a graph showing Luc activity (total RFU) in a spleen in a case of administration using the 30G-pNFI-25/40, the 30G-pNFI-35/40, and the 30G needle-equipped Hamilton syringe (30G-Syr/pump). Mean + SD.
FIG. 12 is a graph showing Luc activity (total RFU) in a testis in a case of administration using the 30G-pNFI-25/40, the 30G-pNFI-35/40, and the 30G needle-equipped Hamilton syringe (30G-Syr/pump). Mean + SD.
FIG. 13 is a graph showing Luc activity (total RFU) in an epididymal head in a case of administration using the 30G-pNFI-25/40, the 30G-pNFI-35/40, and the 30G needle-equipped Hamilton syringe (30G-Syr/pump). Mean + SD.
FIG. 14 is a graph showing Luc activity (total RFU) in an epididymal tail in a case of administration using the 30G-pNFI-25/40, the 30G-pNFI-35/40, and the 30G needle-equipped Hamilton syringe (30G-Syr/pump). Mean + SD.
FIG. 15 is a graph showing Luc activity (total RFU) in a liver in a case of administration using the 30G-pNFI-25/40 and the 30G needle-equipped insulin syringe (30G-Syr). Mean + SD.

### Description of Embodiments

Each of the configurations, combinations thereof, and the like in each of the embodiments is an example, and various additions, omissions, substitutions, and other changes of the configurations may be made as appropriate without departing from the spirit of the present disclosure. The present disclosure is not limited by the embodiments and is limited only by the claims. Each aspect disclosed in the present specification can be combined with any other feature disclosed herein.

Further, a numerical range expressed using "to" denotes a range including the numerical values described before and after "to" as the lower limit value and the upper limit value. Specifically, "A to B" denotes a range of A or more and B or less.

In an embodiment of the present disclosure, an injector for injecting a solution containing a biofunctional substance into an injection target includes:
an accommodation part configured to accommodate the solution containing the biofunctional substance;
a pressurization part configured to pressurize the solution containing the biofunctional substance during actuation; and
an ejection part insertable into the injection target and including
an inlet port configured to facilitate inflow of the solution containing the biofunctional substance from the accommodation part when the solution containing the biofunctional substance is pressurized by the pressurization part, and
an outlet port configured to facilitate ejection of the flowing solution containing the biofunctional substance into the injection target. An ejection rate of the solution containing the biofunctional substance exceeds 83.3 µL/s.

The injector of the present embodiment enables high physiological activity when a solution containing a biofunctional substance exhibiting physiological activity in an injection target is injected into the injection target.

### Solution Containing Biofunctional Substance

The solution that can be injected by the injector according to the present embodiment contains a biofunctional substance. The biofunctional substance is not particularly limited as long as the substance exhibits physiological activity in the injection target. The solution may contain a single type of the biofunctional substance or two or more types thereof. The biofunctional substance may be a natural product or may be an artificially synthesized product.

The biofunctional substance is preferably one or more selected from the group consisting of a nucleic acid, a peptide, a protein, and a low-molecular-weight compound.

Examples of the nucleic acid include deoxyribonucleic acid (DNA), ribonucleic acid (RNA), and peptide nucleic acid (PNA). The nucleic acid may be a nucleic acid including a portion encoding a protein, or a nucleic acid not including a portion encoding a protein (non-coding nucleic acid).

Examples of the peptides and the proteins include antigens (that is, those that produce antibodies against the peptides and proteins), antibodies, peptide vaccines, protein vaccines, peptide hormones, protein hormones, growth factors, cytokines, blood coagulation factors, serum albumin, digestive enzymes, anti-inflammatory peptides, and anti-inflammatory proteins.

A low-molecular-weight compound typically refers to a compound having a molecular weight of 2000 or less. However, the low-molecular-weight compound is not limited thereto, and encompasses a compound that can be treated as a low-molecular-weight compound in the industry. Examples of a preferable range of the molecular weight of the low-molecular-weight compound include 50 or greater, or 100 or greater. Further examples include 2000 or less and 1000 or less. That is, examples include 50 to 2000, 50 to 1000, and 100 to 2000.

In the present disclosure, physiological activity refers to an effect on a specific physiological regulatory function of a living body. An evaluation index of the physiological activity can be set as appropriate according to purpose, and may be either a qualitative index or a quantitative index, but a quantitative index is preferred. For example, a specific mRNA amount, protein amount, cytokine amount, antibody titer, or the number of cells of a specific cell type can be used as an index. These quantitative indices can be quantified by methods known in the art.

In particular, when the biofunctional substance is a nucleic acid containing a portion encoding a protein, an amount of the protein encoded by the nucleic acid can be quantified and used as an index for evaluating the physiological activity. Further, the activity of the protein may be quantitatively evaluated. For example, as illustrated in examples described below, when the protein is luciferase, the physiological activity can be evaluated by measuring an intensity of bioluminescence.

When the biofunctional substance is a nucleic acid, the nucleic acid may be incorporated into a virus vector or carried by a lipid nanoparticle and contained in the solution. However, according to the injector of the present embodiment, high physiological activity can be achieved even when a virus vector or a lipid nanoparticle is not used. When a virus vector or a lipid nanoparticle is not used, it is possible to reduce the possibility that a side reaction such as anaphylaxis will be induced in the injection target.

A content of the biofunctional substance relative to the total amount of the solution can be set as appropriate on the basis of the type of the biofunctional substance, the injection target, the physiological activity exhibited by the biofunctional substance in the injection target into which the biofunctional substance is injected, and the like.

In addition to the biofunctional substance, the solution may contain common additives such as a buffer, a tonicity agent, a pH adjuster, an antioxidant, a thickener, a stabilizer, a wetting agent, an emulsifier, and a binder, as necessary.

As the buffering agent, various types can be used, such as phosphate-based buffers (for example, phosphate buffer, phosphate buffered saline (PBS) (which may be PBS(+) or PBS(-)), Dulbecco's phosphate buffered saline (D-PBS), citrate-phosphate buffer, citrate-phosphate buffered saline), citrate buffers, tris(hydroxymethyl)aminomethane-HCl buffers (Tris-HCl buffer), acetate buffers, GOOD buffers (for example, HEPES-NaOH buffer), amino acid-based buffers (for example, glycine-HCl buffer, glycine-NaOH buffer, glycylglycine-NaOH buffer, glycylglycine-KOH buffer), and imidazole buffers.

Among these, phosphate-based buffers are preferable from the viewpoint of versatility.

Examples of the tonicity agent include an ionic tonicity agent and a nonionic tonicity agent.

Examples of the ionic tonicity agent include salts such as sodium chloride, potassium chloride, calcium chloride, and magnesium chloride.

Examples of the nonionic tonicity agent include glycerin, propylene glycol, polyethylene glycol, glucose, sorbitol, mannitol, trehalose, maltose, and sucrose.

Among these, sodium chloride is preferable from the viewpoint of versatility.

Examples of the pH adjuster include hydrochloric acid, phosphoric acid, citric acid, acetic acid, sodium hydroxide, potassium hydroxide, sodium carbonate, and sodium hydrogen carbonate.

Examples of the antioxidant include ascorbic acid, sodium sulfite, butylhydroxyanisole, butylhydroxytoluene, propyl gallate, and tocopherol.

Examples of the thickener include alginic acid, polyethylene glycol, hydroxypropylmethyl cellulose, and sodium carboxymethyl cellulose.

The content of the biofunctional substance in the solution is not particularly limited, and can be adjusted as appropriate by a person skilled in the art within a range in which the biofunctional substance can exhibit physiological activity. Examples of the content include 0.0001 mg/mL or higher, 0.001 mg/mL or higher, and 0.01 mg/mL or higher. Further, examples include 1000 mg/mL or less and 100 mg/mL or less. That is, examples include 0.0001 to 1000 mg/mL, 0.001 to 100 mg/mL, and 0.01 to 100mg/mL.

The pH of the solution is not particularly limited as long as, when the solution is injected into the injection target, the biofunctional substance exhibits physiological activity and is stably present in the injection target, and there is no adverse effect such as destruction of the injection target.

### Injector

As described above, the injector according to the present embodiment includes:
the accommodation part that accommodates the solution containing the biofunctional substance;
the pressurization part that pressurizes the solution; and
the ejection part insertable into the injection target of the solution.

The injector injects the solution into the injection target by ejecting the solution from the outlet port of the ejection part.

In the injector according to the present embodiment, application of energy by the pressurization part for pressurizing the solution containing the biofunctional substance can adopt a form of energy application based on a known pressurization technique. An example of the energy applied may be a chemically-generated energy such as, for example, a combustion energy generated by an oxidation reaction of a low explosive or a high explosive. Further, as another method, the energy for pressurization may be generated electrically. As one example, energy caused by a piezoelectric element or an electromagnetic actuator driven by applied electric power may be employed. Furthermore, as yet another method, the energy for pressurization may be generated physically. As one example, elastic energy of an elastic body or internal energy of a compressed body, such as compressed gas, may be employed. That is, the energy for pressurization may be any energy as long as the energy enables ejection of the solution with the injector. Further, the energy for pressurizing the solution may be a composite-type energy obtained by appropriately combining the above-described combustion energy, energy generated by electric power, and internal energy such as elastic energy.

From the above, for example, the pressurization part may perform pressurization by utilizing pressure generated by combustion of the explosive ignited by the ignition device, or may perform pressurization by utilizing pressure generated when the compressed gas is released. Further, the pressurization part may apply pressure by utilizing a biasing force of a compression spring, or may apply pressure by utilizing an electromagnetic force such as, for example, a linear electromagnetic actuator. The pressurization part preferably utilizes at least the pressure generated by the combustion of the explosive ignited by the ignition device, and may be used in combination with any of the other pressurization modes described above.

In a case in which the pressurization part utilizes the pressure generated by combustion of an explosive, the explosive used may be, for example, any one of an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), and an explosive containing aluminum and iron oxide (AFO), or an explosive composed of two or more of these explosives in combination. Such an explosive is characterized in that the combustion product thereof is gas at a high temperature but does not contain a gas component at normal temperature, and hence, the combustion product is condensed immediately after the ignition.

Further, in a case in which the pressurization part utilizes energy generated by combustion of a gas generating agent as ejection energy, a single base smokeless explosive (GG) and various types of gas generating agents used in a gas generator for an air bag and a gas generator for a seat belt pretensioner may be used as the gas generating agent.

The injector according to the present embodiment can be configured as, for example, a needle-equipped injector including:
an injector body including an accommodation part and a pressurization part; and
an injection needle attached to the injector body as an ejection part. Further, in the injector according to the present embodiment, the accommodation part and the ejection part of the injector body may be integrally molded. Further, the injector body may be configured as, for example, a needleless injector capable of injection by itself without using an injection needle. However, the injector body according to the present disclosure is not limited to a needleless injector. The injector body may be, for example, a syringe pump. Further, the ejection part according to the present disclosure is not limited to an injection needle. For example, in a case in which a distance from the injector body to the injection target is long or the like, a catheter or a multi-hole injection needle may be used as the ejection part and facilitate guidance of the solution containing the biofunctional substance from the accommodation part of the injector body to the injection target.

Here, from the viewpoint of suppressing tissue damage of the injection target, the ejection part inserted into the injection target is preferably thin. Specifically, an inner diameter of the ejection part is preferably 0.41 mm or less, and an outer diameter of the ejection part is preferably 0.72 mm or less. However, ranges of the inner diameter and the outer diameter of the ejection part according to the present disclosure are not limited to the above. Further, a length of the ejection part can be set as appropriate in accordance with the injection target. For example, in a case in which the ejection part is constituted by an injection needle, the injection needle may have a length in a range from 1 mm to 180 mm. As long as the length of the injection needle is 1 mm or longer, the injection needle can reach the inside of the skin when the injection needle is vertically inserted into the skin. Further, even in a case in which a deep portion of a human internal organ is assumed to be the injection target, the injection needle having a length of 180 mm is enough to reach the deep portion of the internal organ. Further, a material of the ejection part is not particularly limited, but a metal material or a resin material can be adopted.

In the injector according to the present embodiment, the solution containing the biofunctional substance may be accommodated in the accommodation part from the start, or may be accommodated in the accommodation part by being suctioned from outside the injector through the outlet port of the ejection part.

### Injector

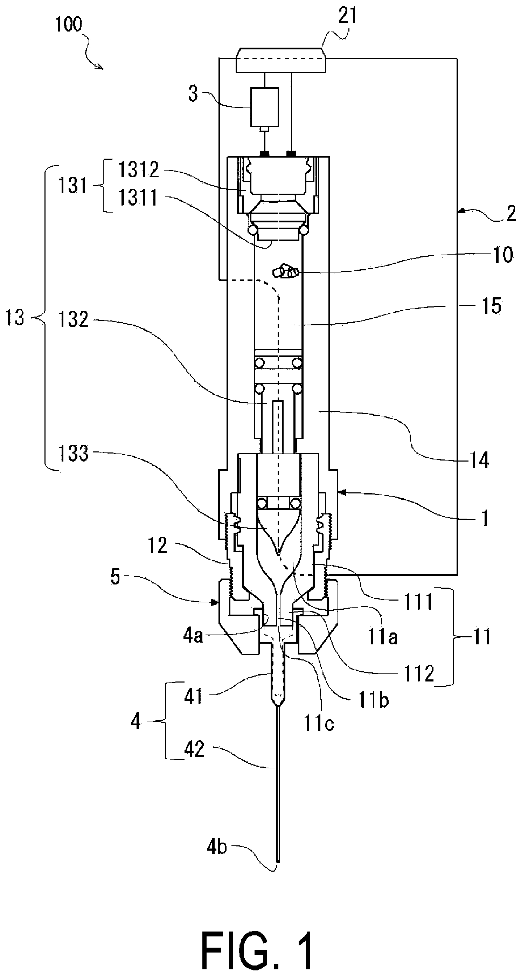

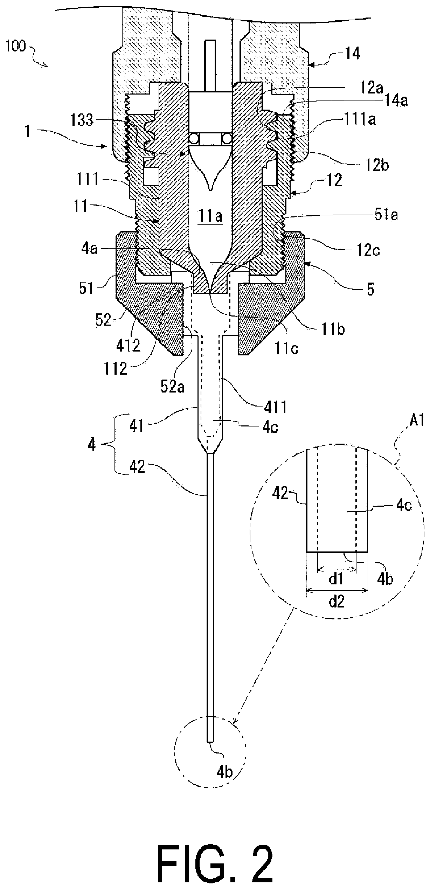

With reference to the drawings, an injector 100 illustrated in FIGS. 1 and 2 will be described below as an example of the injector according to the present embodiment. Note that configurations of the following embodiment are provided as examples, and the technique of the present disclosure is not limited to the configurations of this embodiment. Note that, in the following description, the terms "tip end side" and "base end side" are used as terms indicating a relative positional relationship in a longitudinal direction of the injector 100. The "tip end side" refers to an outlet port 4b side illustrated in FIG. 1 and the like in the longitudinal direction of the injector 100, and the "base end side" refers to a side opposite to the outlet port 4b side in the longitudinal direction of the injector 100.

FIG. 1 is an overall view of the injector 100 according to the present embodiment. FIG. 1 illustrates a cross section along the longitudinal direction of the injector 100. Further, FIG. 2 is a cross-sectional view illustrating the vicinity of a tip end portion of the injector 100. As illustrated in FIG. 1, the injector 100 includes a jet injector 1 as an injector body, a housing 2 that accommodates the jet injector 1, an injection needle 4 as an ejection part, and a fixing jig 5 for fixing the injection needle 4 to the jet injector 1.

### Jet Injector

As illustrated in FIG. 1, the jet injector 1 includes a container 11, a container holder 12, an actuator 13, and a casing 14, which are integrally assembled to form an assembly. An ejection port denoted by reference sign 11c is formed at a tip end of the jet injector 1. The jet injector 1 pressurizes the solution containing the biofunctional substance (hereinafter, also simply referred to as a "solution") utilizing the combustion energy of an explosive, thereby ejecting the solution from the ejection port 11c. The jet injector 1 according to the present embodiment is configured as a needleless injector capable of injection by itself without using an injection needle. However, the jet injector 1 need not be a needleless injector.

### Casing

As illustrated in FIG. 1, the casing 14 is a tubular member accommodating the actuator 13. An ignition device 131 (described below) of the actuator 13 is fitted into the base end side of the casing 14, and the container holder 12 is fitted into the tip end side of the casing 14. As illustrated in FIG. 2, a threaded part 14a for coupling the casing 14 and the container holder 12 is formed on an inner circumferential surface of the tip end side of the casing 14. The container holder 12 and the actuator 13 are coupled via the casing 14.

### Container

As illustrated in FIG. 2, the container 11 includes a main body part 111 and a nozzle part 112 having a diameter smaller than that of the main body part 111, and is formed in a tubular shape. Further, inside the container 11 are formed an accommodating space 11a that is a space capable of accommodating the solution containing the biofunctional substance, and a flow path 11b that communicates with the accommodating space 11a and opens on the tip end side. More specifically, the accommodating space 11a is formed inside the main body part 111, and the flow path 11b is formed inside the nozzle part 112. The accommodating space 11a is an example of an accommodation part according to the present disclosure. An opening of the flow path 11b is formed in a tip end surface of the nozzle part 112, and the opening serves as the ejection port 11c of the jet injector 1. That is, the jet injector 1 ejects the solution from the ejection port 11c formed in the tip end surface of the nozzle part 112. As illustrated in FIG. 2, an inner diameter of the flow path 11b of the container 11 is smaller than an inner diameter of the accommodating space 11a. With such a configuration, the solution pressurized to a high pressure is ejected to the outside through the ejection port 11c of the flow path 11b. Further, a threaded part 111a for coupling the container 11 and the container holder 12 is formed on an outer circumferential surface of the main body part 111.

A material of the container 11 is not particularly limited. For example, the container 11 can be formed of a resin material. As the resin material forming the container 11, examples of materials that can be used include known materials such as nylon 6-12, polyarylate, polycarbonate, polybutylene terephthalate, polyphenylene sulfide, or liquid crystal polymer.

### Container Holder

As illustrated in FIG. 2, the container holder 12 is formed in a tubular shape and holds the container 11 fitted into the container holder 12. A threaded part 12a is formed on an inner circumferential surface of the base end side of the container holder 12. The threaded part 111a of the container 11 and the threaded part 12a of the container holder 12 are screwed together, thereby coupling the container 11 and the container holder 12. Further, a threaded part 12b is formed on an outer circumferential surface of the base end side of the container holder 12. The threaded part 12b of the container holder 12 and the threaded part 14a of the casing 14 are screwed together, thereby coupling the container holder 12 and the casing 14. Further, a threaded part 12c for coupling the container holder 12 and the fixing jig 5 is formed on the outer circumferential surface of the tip end side of the container holder 12.

A material of the container holder 12 is not particularly limited. For example, the container holder 12 can be formed of a metal material. Examples of the metal material forming the container holder 12 include stainless steel, copper, aluminum, iron, titanium, and a titanium alloy.

### Actuator

The actuator 13 illustrated in FIG. 1 is configured to pressurize the solution when actuated. The actuator 13 is an example of a "pressurization part" according to the present disclosure. As illustrated in FIG. 1, the actuator 13 includes the ignition device 131 and a piston 132 accommodated in the casing 14, and a plunger 133.

The ignition device 131 is disposed on the base end side of the casing 14, the plunger 133 is disposed on the tip end side of the casing 14, and the piston 132 is disposed adjacent to the plunger 133 and between the ignition device 131 and the plunger 133. A combustion chamber 15 is formed between the ignition device 131 and the piston 132 in an internal space of the casing 14.

### Ignition Device

The ignition device 131 includes an initiator 1311 and a holding member 1312. The initiator 1311 serves as a drive source of the actuator 13 and generates energy for pressurization and ejection of the solution by the injector 100. The initiator 1311 is configured as an electric igniter that releases a combustion product by burning an ignition agent accommodated in an interior thereof. The holding member 1312 is formed by injection molding of a resin. The injection molding can be performed by a known method. Further, for the holding member 1312, a resin material similar to that of the container 11 can be used. The ignition device 131 is configured as an igniter assembly in which the initiator 1311 is fixed to a base end portion of the casing 14 via the holding member 1312, and is fitted into the casing 14, closing the base end portion of the casing 14. Further, the ignition device 131 is disposed in the casing 14 with the initiator 1311 facing a base end surface (end surface on the base end side) of the piston 132, and thus the combustion energy of the ignition agent by the initiator 1311 and the combustion energy of a gas generating agent 10 described below are transmitted to the base end surface.

### Ignition Agent

Here, examples of the ignition agent used for the initiator 1311 include an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), an explosive containing aluminum and ferric oxide (AFO), and an explosive composed of a combination of two or more of these explosives. These explosives exhibit characteristics that, although the explosives generate high-temperature and high-pressure plasma during combustion immediately after ignition, when the combustion product condenses at room temperature, the explosives do not contain gaseous components and hence the pressure generated decreases abruptly. Note that an explosive other than these may be used as the ignition agent as long as appropriate ejection of the solution can be performed.

### Gas Generating Agent

In the jet injector 1, to adjust the transition of the pressure applied to the solution via the piston 132, the gas generating agent 10 burned by the combustion product of the ignition agent released from the initiator 1311 to generate gas is disposed in the combustion chamber 15. That is, the jet injector 1 is configured to pressurize the solution by utilizing the combustion energy of the gas generating agent 10 in addition to the combustion energy of the ignition agent by the initiator 1311. The gas generating agent 10 is disposed at a location that can be exposed to the combustion product from the initiator 1311. Alternatively, the gas generating agent 10 may be disposed in the initiator 1311 as disclosed in WO 01/031282, JP 2003-25950 A, and the like. One example of the gas generating agent may be exemplified by a single base smokeless explosive (GG) formed of 98 mass% of nitrocellulose, 0.8 mass% of diphenylamine, and 1.2 mass% of potassium sulfate. Various types of gas generating agents used in a gas generator for an air bag and a gas generator for a seat belt pretensioner may be used. A combustion completion time period of the gas generating agent can be changed by adjusting a dimension, a size, and a shape, particularly, a surface shape, of the gas generating agent at the time of being disposed in the combustion chamber 15. With this, the transition of the pressure applied to the solution is adjusted, and an ejection pressure can be transitioned in a desired manner. Note that the jet injector 1 need not include the gas generating agent 10 and may pressurize the solution by utilizing only the combustion energy of the ignition agent by the initiator 1311. In the present disclosure, the gas generating agent and the like to be used as necessary are also included in the pressurization part.

### Piston

The piston 132 is disposed on the tip end side of the casing 14, facilitating sliding inside the casing 14 by being pressurized by actuation of the initiator 1311. The piston 132 is formed of a metal, and an O-ring or the like may be disposed on a portion thereof to enhance sealing with a sliding surface (that is, inner circumferential surface of the casing 14) on which the piston 132 slides. Alternatively, the piston 132 may be formed of a resin and, in such a case, a metal may be used in combination for a portion requiring heat resistance and pressure resistance.

### Plunger

The plunger 133 is a member that receives the combustion energy of the ignition agent by the initiator 1311 and the combustion energy of the gas generating agent via the piston 132, thereby pressurizing the solution accommodated in the accommodating space 11a. The plunger 133 is accommodated in the casing 14 and disposed between the piston 132 and the nozzle part 112 of the container 11. The plunger 133 is formed in a rod shape, and a base end portion thereof is engaged with a tip end portion of the piston 132. Further, a tip end portion of the plunger 133 is inserted into the main body part 111 of the container 11, and the accommodating space 11a is defined by the plunger 133 and the main body part 111. The plunger 133 is slidable inside the main body part 111 and, with the sliding of the plunger 133, the solution accommodated in the accommodating space 11a is pressurized, passes through the flow path 11b, and is ejected from the ejection port 11c. Thus, the plunger 133 is formed of a material that smoothly slides against the main body part 111 of the container 11 and prevents the solution from leaking from the plunger 133 side.

Specific examples of the material of the plunger 133 include butyl rubber and silicone rubber. Further, for the purpose of securing and adjusting slidability between the plunger 133 and the main body part 111 of the container 11, an outer circumferential surface of plunger 133 and the inner circumferential surface of the main body part 111 may be subjected to coating or surface finishing with various substances.

Here, a contour of a tip end portion of the plunger 133 is formed in a shape substantially coinciding with a contour of a tip end portion of the accommodating space 11a. In this way, in ejection of the solution, when the plunger 133 slides and reaches a deepest position located furthest into the main body part 111, the accommodating space 11a formed between the plunger 133 and the nozzle part 112 can be reduced as much as possible, and the solution can thus be inhibited from remaining in the accommodating space 11a and being wasted.

### Housing

The housing 2 illustrated in FIG. 1 is a member that accommodates the jet injector 1 and functions as a grip part that a user grasps to use the injector 100. As illustrated in FIG. 1, a battery 3 for supplying a drive current to the actuator 13 (more specifically, the initiator 1311) of the jet injector 1 is provided inside the housing 2. Further, as illustrated in FIG. 1, an outer surface of the housing 2 is provided with a plurality of switches 21 for operating the jet injector 1 to achieve ejection of the solution. Further, an inner surface of the housing 2 is provided with a socket (not illustrated) connected to the initiator 1311 of the jet injector 1. Power from the battery 3 to the jet injector 1 is supplied between an electrode on the housing 2 side and an electrode on the initiator 1311 side of the jet injector 1 via wiring by a press operation of the switch 21 by the user. Further, a control unit (not illustrated) such as a microcomputer is provided inside the housing 2. The control unit controls supply of an ignition current to the initiator 1311 of the jet injector 1 on the basis of a signal from each switch, thereby performing operation control of the jet injector 1.

Note that, as described above, in the present embodiment, the power for actuating the initiator 1311 is supplied from the battery built into the housing 2. However, instead of this mode, the power may be supplied from the outside via a power cable.

### Injection Needle

As illustrated in FIG. 1, the injection needle 4 is attached to the nozzle part 112 of the jet injector 1 and includes a base section 41 and a needle tube 42. The injection needle 4 is an example of an "ejection part" according to the present disclosure, and is configured to facilitate inflow of the solution pressurized by the actuator 13 of the jet injector 1 and ejection of the flowing solution to the injection target.

The solution pressurized by the actuator 13 flows into the base section 41. As illustrated in FIG. 1, the base section 41 is formed in a tubular shape. More specifically, as illustrated in FIG. 2, the base section 41 includes a base body 411 having a tubular shape and a flange part 412 formed at a base end portion of the base body 411 and protruding outward in a radial direction. The nozzle part 112 of the jet injector 1 is press-fitted into an opening on the base end side of the base body 411. Accordingly, the opening on the base end side of the base body 411 is configured as an inlet port 4a through which the solution containing the biofunctional substance can flow from the accommodating space 11a of the container 11 into the injection needle 4.

The base section 41 can be formed of, for example, a resin material. As the resin material forming the base section 41, a known synthetic resin such as polycarbonate, polypropylene, or polyethylene, for example, can be adopted. Further, the base section 41 may be made of a metal. Examples of the metal material forming the base section 41 include stainless steel, aluminum, an aluminum alloy, titanium, and a titanium alloy. The material of the base section 41 is not particularly limited, but in consideration of suppression of pressure loss of the solution flowing into the injection needle 4, a material having relatively high rigidity such as stainless steel is preferably used for the base section 41.

The needle tube 42 is inserted into the injection target and ejects the solution containing the biofunctional substance into the injection target. A base end portion of the needle tube 42 is connected to a tip end of the base section 41, and an internal space of the base section 41 and an internal space of the needle tube 42 communicate with each other. Accordingly, an opening on the tip end side of the needle tube 42 is configured as the outlet port 4b through which the solution containing the biofunctional substance flowing into the injection needle 4 from the inlet port 4a can be ejected to the injection target. Further, a sharp needle tip that punctures the skin is formed at a tip end portion of the needle tube 42, facilitating insertion of the needle tube 42 into the injection target.

Here, from the viewpoint of suppressing tissue damage of the injection target, the injection needle 4 (more specifically, the needle tube 42 inserted into the injection target) is preferably thin. As illustrated in an enlarged view A1 of FIG. 2, an inner diameter of the needle tube 42 is defined as d1, and an outer diameter of the needle tube 42 is defined as d2. At this time, the inner diameter d1 is preferably 0.41 mm or less, and the outer diameter d2 is preferably 0.72 mm or less. However, the ranges of the inner diameter and the outer diameter of the needle tube 42 are not limited to the above. Further, a length of the needle tube 42 can be set as appropriate in accordance with the injection target. For example, a value in a range from 1 mm to 180 mm can be selected and used.

A material of the needle tube 42 is not particularly limited, but examples thereof include stainless steel. Further, as a metal material other than stainless steel, aluminum, an aluminum alloy, titanium, a titanium alloy, or the like can be adopted. Further, the needle tube 42 may be formed of a resin material.

As described above, the internal space of the base section 41 and the internal space of the needle tube 42 communicate with each other, and thus, in the injection needle 4, a flow path 4c from the inlet port 4a to the outlet port 4b is formed by the internal space of the base section 41 and the internal space of the needle tube 42. The solution flowing into the injection needle 4 from the inlet port 4a flows through the flow path 4c, reaches the outlet port 4b, and is ejected from the outlet port 4b to the injection target.

A sealant may be used between the nozzle part 112 of the container 11 and the base section 41 of the injection needle 4 to improve liquid-tightness. Examples of the sealant include an O-ring, packing, sealing tape, and a liquid sealant.

### Fixing Jig

The fixing jig 5 is a member for fixing the injection needle 4 to the nozzle part 112 of the jet injector 1. The fixing jig 5 includes a surrounding wall part 51 having a tubular shape and a lid wall part 52 that closes a tip end of the surrounding wall part 51. The surrounding wall part 51 is fitted into the container holder 12. Further, a threaded part 51a is formed on an inner circumferential surface of the surrounding wall part 51, and the container holder 12 and the fixing jig 5 are coupled by screwing the threaded part 12c of the container holder 12 and the threaded part 51a of the fixing jig 5 to each other. The lid wall part 52 is formed with a through hole 52a passing through the lid wall part 52 from the base end side to the tip end side. The base body 411 of the injection needle 4 is fitted into the through hole 52a. At this time, the lid wall part 52 presses the flange part 412 of the injection needle 4 from the tip end side, thereby preventing the injection needle 4 from falling off the nozzle part 112 of the jet injector 1. In this way, the injection needle 4 is fixed to the nozzle part 112 by the fixing jig 5.

A material of the fixing jig 5 is not particularly limited, but the fixing jig 5 can be formed of a metal material such as stainless steel, copper, aluminum, iron, titanium, or a titanium alloy, for example, as with the container holder 12. Further, for example, the fixing jig 5 may be formed of a resin material such as polycarbonate, polypropylene, or polyethylene.

### Operation of Injector

The injection of the solution into the injection target by the injector 100 is performed by operating the injector 100 with the needle tube 42 of the injection needle 4 being inserted into the injection target. The operation of the injector 100 will be described below. Note that the solution containing the biofunctional substance may be accommodated in the accommodating space 11a of the container 11 from the start, or may be accommodated in the accommodating space 11a by suctioning the solution from outside the injector 100 through the outlet port 4b of the injection needle 4. In a case in which the accommodation part and the ejection part are integrally molded, the solution containing the biofunctional substance is accommodated in the accommodating space 11a of the container 11 from the start, making it possible to reduce medical liquid leakage and dead volumes.

When the drive current is supplied to the actuator 13 of the jet injector 1 (more specifically, the initiator 1311) by operation of the switches 21 by the user and the actuator 13 is actuated, the combustion product of the ignition agent is released from the initiator 1311 to the combustion chamber 15. Thus, the gas generating agent 10 disposed in the combustion chamber 15 is burned by the combustion product of the ignition agent, generating gas in the combustion chamber 15. As described above, the base end surface of the piston 132 is exposed to the combustion chamber 15. Therefore, when the actuator 13 is actuated, the piston 132 receiving the combustion energy (pressure) of the ignition agent or the gas generating agent at the base end surface slides toward the tip end side of the casing 14. Thus, the plunger 133 is pushed toward the tip end side of the accommodating space 11a by the piston 132, and the solution in the accommodating space 11a is pressurized. As a result, the solution passes through the flow path 11b from the accommodating space 11a and is ejected from the ejection port 11c formed in the nozzle part 112.

The solution ejected from the ejection port 11c of the nozzle part 112 flows into the injection needle 4 through the inlet port 4a of the injection needle 4 attached to the nozzle part 112. The solution flows through the flow path 4c and is ejected from the outlet port 4b to the injection target. As described above, the operation of the injector 100 is completed.

### Modified Examples

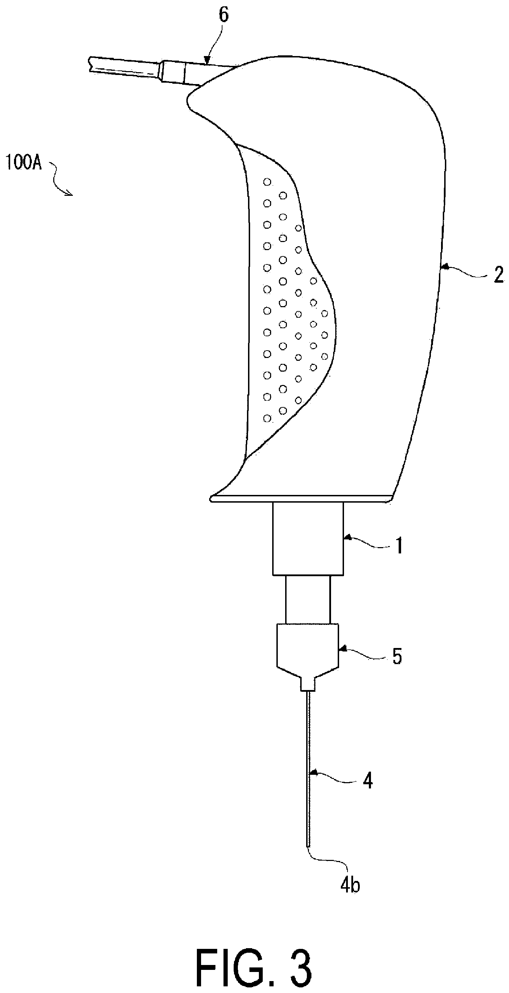

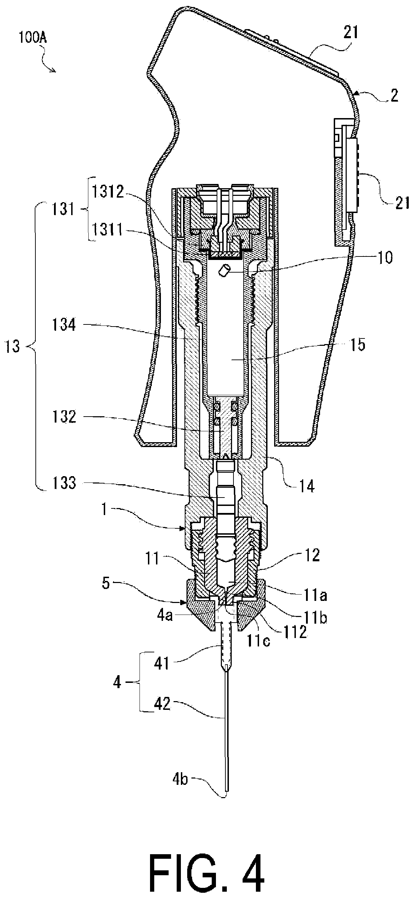

FIG. 3 is an overall view of an injector 100A according to a modified example of the present embodiment. FIG. 3 illustrates an external appearance of the injector 100A. Further, FIG. 4 is a cross-sectional view of the injector 100A. As illustrated in FIG. 3, the housing 2 of the injector 100A is provided with a power cable 6 for supplying a drive current to the actuator 13 of the jet injector 1. Further, as illustrated in FIG. 4, the actuator 13 of the jet injector 1 according to the modified example includes a body 134 having a tubular shape that accommodates the ignition device 131 and the piston 132. The ignition device 131 is disposed on the base end side and the piston 132 is disposed on the tip end side of the body 134. The combustion chamber 15 is formed between the ignition device 131 and the piston 132 in an internal space of the body 134. The injector according to the present disclosure can also be implemented with a configuration such as that of the injector 100A.

### Ejection Rate of Solution

The injector according to the present embodiment can eject the solution containing the biofunctional substance at an ejection rate exceeding 83.3 µL/s. The ejection rate is preferably 200 µL/s or higher, more preferably 250 µL/s or higher, even more preferably 300 µL/s or higher, even more preferably 350 µL/s or higher, even more preferably 400 µL/s or higher, and most preferably 500 µL/s or higher. An upper limit thereof is not particularly limited, and may be 5000 µL/s or less. That is, preferable ranges of the ejection rate include higher than 83.3 µL/s and 5000 µL/s or less, from 200 µL/s to 5000 µL/s, from 250 µL/s to 5000 µL/s, from 300 µL/s to 5000 µL/s, from 350 µL/s to 5000 µL/s, from 400 µL/s to 5000 µL/s, and from 500 µL/s to 5000 µL/s.

When the ejection rate is a rate exceeding 83.3 µL/s, high physiological activity can be achieved when the solution is injected into the injection target. Although the detailed mechanism is not clear, presumably when shear stress is applied to the tissues and cells around the administration area, endocytosis is enhanced and delivery of the biofunctional substance into the cells is improved. On the basis of the above-described mechanism, a higher ejection rate in the range described above is considered preferred.

When the ejection rate is less than or equal to the upper limit described above, it is possible to suppress damage to cells and tissues.

In the present disclosure, the ejection rate is the rate of the solution when ejected from the ejection port, and can be calculated by, for example, dividing the ejected volume by the time required from the start to the end of ejection. The time required from the start to the end of the ejection can be measured by, for example, imaging the state of the solution being ejected from the ejection port with an imaging device such as a high-speed camera.

The ejection rate can be set in the range described above by adjusting the ejection energy in accordance with the shape and the material of the ejection part, the volume and a viscosity of the solution, and the like.

### Injection Target

Biological species into which the injector according to the present embodiment can perform injection are not particularly limited, and include mammals. Examples of the mammals include humans, mice, rats, guinea pigs, hamsters, cows, goats, sheep, pigs, monkeys, dogs, and cats. Depending on the case, examples include mammals excluding humans.

The injection target can be the inside a living body, but is not limited thereto. Inside the living body means a portion other than the outer surface of the living body, and may be a surface portion of an organ inside the living body, the inside of an organ, or a space such as a body cavity. Specifically, the injection target is preferably one or more selected from the group consisting of an articular cavity, a spleen, an eyeball, skin, a muscle, a bone, cartilage, marrow, a ligament, a brain, a spinal cord, a lung, a liver, a heart, a kidney, a pancreas, a gall bladder, a digestive tract, a bladder, a reproductive organ, a lymph node, a lymphatic network, a blood vessel, a tumor arising in any one thereof, and mucosa and connective tissue associated with any one thereof. Examples of the reproductive organ include a testis and an ovary. Among these, one or more selected from the group consisting of the articular cavity, the spleen, the testis, and the liver are preferable.

When the injection target is one or more selected from the group consisting of the spleen, the testis, and the liver, a lower limit of the ejection rate of the injector according to the present embodiment may be 1 µL/s or higher, or may be 10 µL/s or higher. Sufficient physiological activity can be achieved by injecting the solution into the injection target at an ejection rate in the range described above.

An injection amount of the solution into the injection target can be set as appropriate on the basis of the content and the type of the biofunctional substance, the injection target, the physiological activity exhibited by the biofunctional substance in the injection target into which the biofunctional substance is injected, and the like.

The injection amount of the solution into the injection target is, for example, 10 µL or more, 50 µL or more, or 100 µL or more. Further, examples include 50 mL or less, 10 mL or less, and 5 mL or less. That is, examples include 10 µL to 50 mL, 50 µL to 10 mL, and 100 µL to 5 mL.

A time required for injecting the solution into the injection target is not particularly limited as long as the injection amount of the solution can be ejected at the ejection rate. Examples of the time include 0.005 seconds or more, 0.01 seconds or more, or 0.1 seconds or more. Further, examples of the time may be 500 seconds or less, 100 seconds or less, and 10 seconds or less. That is, examples include 0.005 to 500 seconds, and 0.01 to 100 seconds, 0.1 to 10 seconds.

### Examples

Hereinafter, the present disclosure will be specifically described with reference to examples. However, the present disclosure is not limited to the following examples.

### Animals

Sprague-Dawley (SD) rats (8 weeks old, male, Sumitomo Life Information Systems Co., Ltd. (SLC)) or Institute of Cancer Research (ICR) mice (8 weeks old, male, SLC) were used.

All surgical procedures and syringe or device administrations were performed under isoflurane (Pfizer) maintenance anesthesia by using a gas anesthesia device (Nacrobit-E, Natsume Seisakusho Co., Ltd.) or under a triple anesthetic mixture.

All animal experiments conducted were approved by the Animal Experiment Review Committees of the facilities where the experiments were performed, either Material Research Center Co., Ltd. (Kawasaki City) or the Institute for Animal Reproduction Foundation (Kasumigaura City).

### Injection of Solution

For administration to rat knee joints, left and right knee joint areas of each rat were shaved and, under isoflurane anesthesia, the needle tip was inserted 5 mm deep, targeting 5 mm below the midpoint of the patella and the proximal tibial epiphysis, and plasmid pGL4 for luciferase (Luc) expression (hereinafter also referred to as pLuc) was administered. The pGL4 was pre-diluted to 1 mg/mL using PBS.

Administration to a mouse spleen, testis, or liver was performed as follows: under the triple anesthetic mixture, the left ventral area, scrotal area, or central abdominal area was shaved, the target organs were exposed through laparotomy under anesthesia, and the pLuc was injected. After confirmation that there was no bleeding from the injection site, the surgical incision was closed by suturing both the muscle and skin layers.

### Injector

For injection, a 30G injection needle-equipped insulin syringe (Becton, Dickinson and Company), a Hamilton syringe (luer-lock type, syringe capacity 50 to 50000 µL, Hamilton Company) fitted with a 30G injection needle (disposable needle, No. 30 Dentronics injection needle, 0.3 x 12 mm, Dentronics Co., Ltd.), and a jet injector (pNFI, refer to FIG. 1) either fitted with or without a 30G injection needle were used. Manual administration using the 30G-Hamilton syringe was performed by slowly pushing the plunger for approximately 1 second (approximately 30 µL/s). Syringe-pump administration using the 30G-Hamilton syringe was performed with a syringe-pump administration rate program (0.25 to 83.3 µL/s). In contrast, the jet injector used ZPP as the ignition agent and GG (containing 98 mass% nitrocellulose, 0.8 mass% diphenylamine, and 1.2 mass% potassium sulfate) as the gas generating agent, which were utilized to administer the solution. The ejection rate of the jet injector was measured by the test described below. Further, as illustrated in FIG. 2, the injection needle 4 was fixed to a tip end of the nozzle part 112 of the jet injector with the fixing jig 5 made of metal. In the following, for example, the jet injector loaded with 35 mg of ZPP and 40 mg of GG will be denoted as "pNFI-35/40", and a jet injector further equipped with a 30G injection needle will be denoted as "30G-pNFI-35/40".

### Collection of Organ Specimens

Dissection and organ specimen collection were performed after each animal was euthanized by cervical dislocation under isoflurane anesthesia, followed by confirmation of death by exsanguination from the carotid artery. Further, as necessary, the triple anesthetic mixture was administered intraperitoneally (by volume per mouse body weight, medetomidine: 0.3 mg/kg, midazolam: 4 mg/kg, butorphanol: 5 mg/kg), and the same procedures as those under isoflurane anesthesia were performed.

For samples obtained by administration to the knee joint, the skin and excess muscle were removed with scissors, the lower limbs were detached from the hip joints, and each limb was collected in a 15-mL tube and cryopreserved. On the Luc activity measurement day, each frozen specimen was thawed, and the knee joints, skin, knee ligaments, and synovium were collected. As for the sampling site of the joint specimen, the tissue of the rat joint was analyzed in advance and a sampling site appropriate for Luc activity measurement was identified. Specifically, excess muscle was removed from the lower limbs, and the areas near the distal femur and proximal tibia were physically crushed with forceps to remove the bone marrow components, and the remaining material was used as a sampling site. The sampling site contains at least cartilage, synovium, cruciate ligaments, joint capsule, and the like.

For samples obtained by administration into the spleen or the testis, the entire organ was collected as one specimen and cryopreserved at -80°C. The epididymal head and tail associated with the testis were each carefully separated from the testis and cryopreserved.

For the liver, a 1-cm square section (about 3-mm thick) centered on the injection site was collected and cryopreserved at -80°C.

### Luc Activity Measurement

Luc activity was measured using a Luc-assay kit (Promega Corporation). The included passive lysis buffer was added in the following amounts: 1 mL for rat knee joint specimens, 1 mL for mouse spleen specimens and mouse testis specimens, 0.3 mL each for mouse epididymal head and tail specimens, and 0.5 mL for mouse liver specimens. The specimens were then finely cut with scissors. Subsequently, the specimens were frozen, thawed at room temperature, and centrifuged at 10000 × g for 10 minutes at 16°C. Following the protocol, 20 µL of supernatant and 100 µL of substrate solution were mixed in a Lumi Tube (Kikkoman Corporation), and relative fluorescence units (RFUs) were promptly measured using a Lumitester C-110 (Kikkoman Corporation). The total RFU per tissue specimen was calculated by multiplying the RFU measurement value by a correction factor to obtain the RFU per total volume of added passive lysis buffer.

### Test Example 1

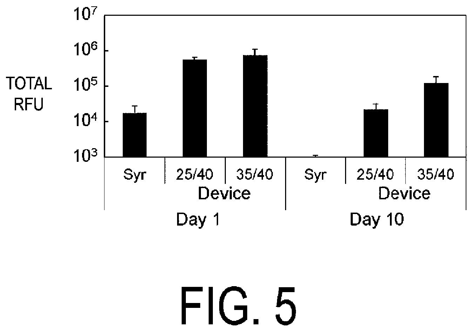

The pLuc with a concentration of 1 mg/mL was administered into the rat knee joint at a volume of 30 µL using a 30G needle-equipped 2.5-mL Hamilton syringe (administration rate of 15 µL/s or 0.5 µL/s; n = 2 each), the 30G-pNFI-25/40 (n = 4), or the 30G-pNFI-35/40 (n = 4). Hereinafter, these are referred to as a Syr group, a 25/40 device group, and a 35/40 device group, respectively. The lower limbs were collected 24 hours (Day 1) and 10 days (Day 10) after administration, and the Luc activity in the knee joints was measured. The results comparing the means + SD are shown in FIG. 5. In the Syr group, the Luc activity (total RFU) values were 14150 and 34350 for 15 µL/s on Day 1, and 11550 and 11800 for 0.5 µL/s on Day 1; and 400 and 400 for 15 µL/s on Day 10, and 1450 and 400 for 0.5 µL/s on Day 10. Given the similar values, the specimens collected at each time point were combined, resulting in the Syr group (n = 4).

The Luc activity values on Day 1 of the 25/40 device group and the 35/40 device group, as compared with that of the Syr group, were 31.1 times higher and 42.3 times higher, respectively (FIG. 5). The Luc activity values on Day 10 of the 25/40 device group and the 35/40 device group, as compared with that of the Syr group, were 32.8 times higher and 184.3 times higher, respectively (FIG. 5). In all groups, as compared with Day 1, a reduction in Luc activity on Day 10 was recognized, but the reduction in the 35/40 device group was small as compared with those of the Syr group and 25/40 device group. On both Day 1 and Day 10, no significant difference was recognized between the 25/40 device group and the 35/40 device group, but a trend towards higher Luc activity in the 35/40 Device group was observed.

### Test Example 2

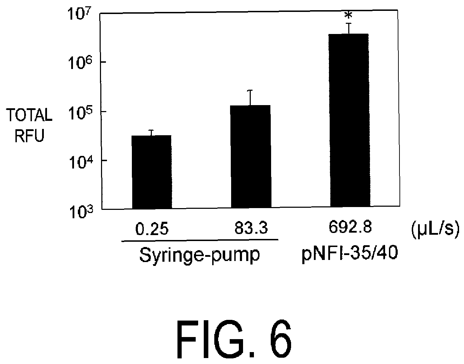

The pLuc with a concentration of 1 mg/mL was administered into the rat knee joint at a volume of 30 µL using a 30G needle-equipped 25-mL Hamilton syringe (administration rate of 0.25 µL/s, n = 4 or 83.3 µL/s, n = 8) or the 30G-pNFI-35/40 (n = 8). Hereinafter, these are referred to as an administration rate 0.25 µL/s group, an administration rate 83.3 µL/s group, and a 30G-pNFI-35/40 group, respectively. Note that the ejection rate of the 30G-pNFI-35/40 group was 692.8 µL/s as shown in Test Example 4 described below. The lower limbs were collected 24 hours after administration, and the Luc activity in the knee joint was measured. The results comparing the means + SD are shown in FIG. 6.

The Luc activity values of the administration rate 83.3 µL/s group and the 30G-pNFI-35/40 group, as compared with that of the administration rate 0.25 µL/s group, were 3.9 times higher and 105 times higher, respectively. The Luc activity of the 30G-pNFI-35/40 group was significantly higher than those of the 0.25 µL/s group and the 83.3 µL/s group.

### Test Example 3

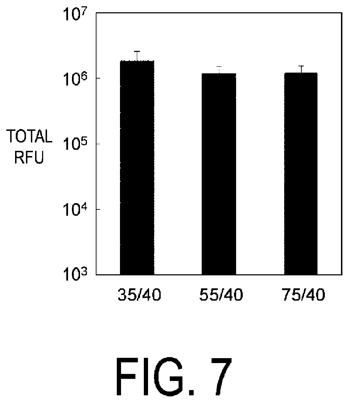

The pLuc with a concentration of 1 mg/mL was administered into the rat knee joint at a volume of 30 µL using the 30G-pNFI-35/40 (n = 6), the 30G-pNFI-55/40 (n = 6), or the 30G-pNFI-75/40 (n = 6). Hereinafter, these are referred to as a 35/40 group, a 55/40 group, and a 75/40 group, respectively. The lower limbs were collected 24 hours after administration, and the Luc activity in the knee joint was measured. The results comparing the means + SD are shown in FIG. 7.

No significant difference was found in the Luc activity of the joints between the 35/40 group, the 55/40 group, and the 75/40 group.

### Test Example 4

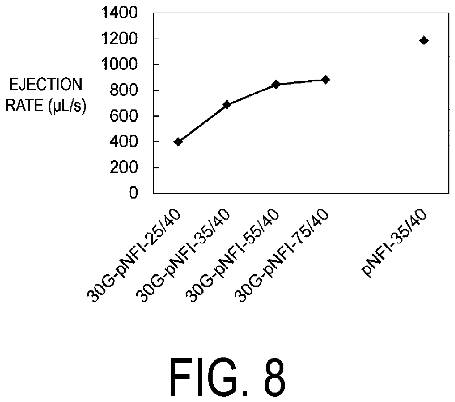

The ejection rates were measured using the 30G-pNFI-25/40, the 30G-pNFI-35/40, the 30G-pNFI-55/40, the 30G-pNFI-75/40, and the pNFI-35/40. The tip end of the injector was immersed in PBS, and 30 µL of 1% malachite green solution was ejected. The time required from the start to the end of ejection was measured by high-speed camera analysis, and each ejection rate was calculated by dividing 30 µL by this time period. The results are shown in FIG. 8.

The ejection rates of the needle-equipped 30G-pNFI were significantly higher than the maximum speed of the syringe pump (83.3 µL/s) under all conditions, measuring 398.4 µL/s or higher. Further, the ejection rate increased in a ZPP dose-dependent manner.

### Test Example 5

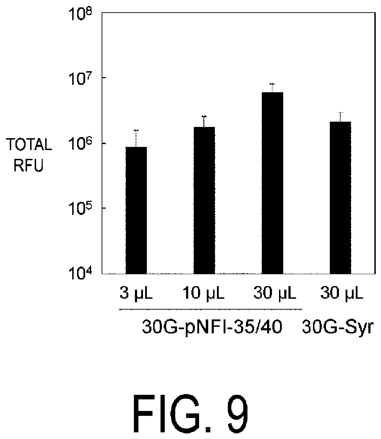

The pLuc with a concentration of 1 mg/mL was administered into the mice spleen using a 30G needle-equipped insulin syringe (administration volume: 30 µL, administration rate: 30 µL/s, n = 4) or the 30G-pNFI-35/40 (administration volume 3 µL: n = 4, 10 µL: n = 4, 30 µL: n = 3). Each spleen was collected 24 hours after administration, and the Luc activity was measured. The results comparing the means + SD are shown in FIG. 9.

When the 30G-pNFI-35/40 was used, the Luc activity increased in a dose-dependent manner. Further, when the same amount of pLuc was administered using the 30G-pNFI-35/40, the Luc activity increased about three-fold as compared with that when the 30G injection needle-equipped insulin syringe was used.

### Test Example 6

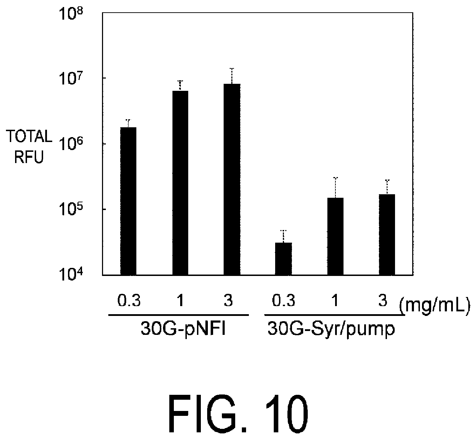

The pLuc with a concentration of 0.3, 1, or 3 mg/mL was administered into the mouse spleen at a volume of 30 µL using a 30G needle-equipped Hamilton syringe (administration rate of 0.25 µL/s, n = 4) or the 30G-pNFI-35/40 (n = 4). Each spleen was collected 24 hours after administration, and the Luc activity was measured. The results comparing the means + SD are shown in FIG. 10.

When the 30G-pNFI-35/40 was used, the Luc activity of the 1 mg/mL administration group was 3.6 times higher than that of the 0.3 mg/mL administration group, while the Luc activity of the 3 mg/mL administration group was about the same as that of the 1 mg/mL administration group. A similar trend was recognized when using the 30G needle-equipped Hamilton syringe (30G-Syr/pump). When the 30G-pNFI-35/40 and the 30G needle-equipped Hamilton syringe having the same pLuc concentrations were compared, the 30G-pNFI-35/40 showed approximately 50 times higher Luc activity than those of the 30G needle-equipped Hamilton syringe across all pLuc concentrations.

### Test Example 7

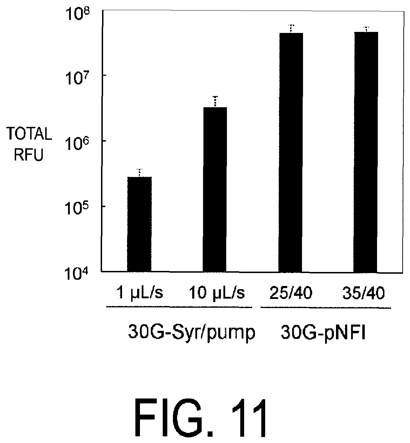

The pLuc with a concentration of 1 mg/mL was administered into the mouse spleen at a volume of 40 µL using a 30G needle-equipped Hamilton syringe (administration rate of 1 µL/s or 10 µL/s; n = 4 each), the 30G-pNFI-25/40 (n = 4), or the 30G-pNFI-35/40 (n = 4). Each spleen was collected 24 hours after administration, and the Luc activity was measured. The results comparing the means + SD are shown in FIG. 11.

When the 30G-pNFI was used, the Luc activity increased in an administration rate (ZPP explosive amount) dependent manner. Further, for the 30G needle-equipped Hamilton syringe (30G-Syr/pump), the Luc activity increased in an administration rate dependent manner.

### Test Example 8

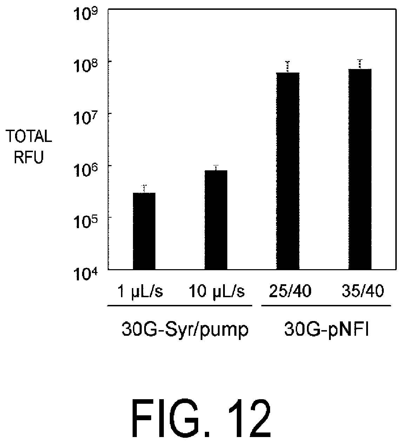

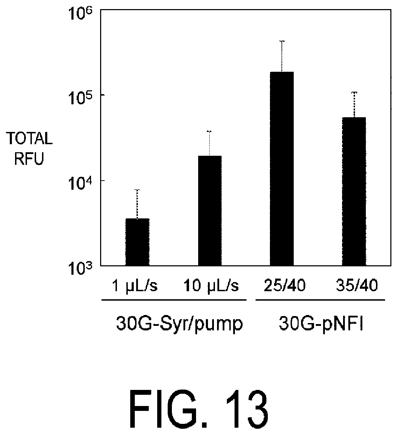

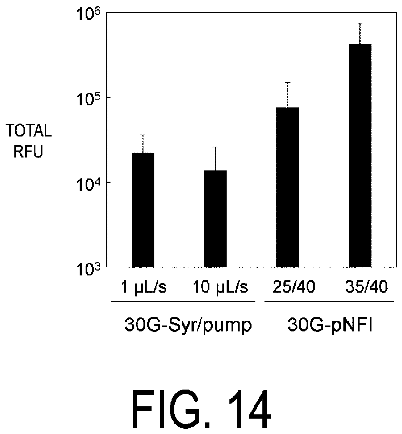

The pLuc with a concentration of 1 mg/mL was administered into the mouse testis at a volume of 30 µL using a 30G needle-equipped Hamilton syringe (administration rate of 1 µL/s or 10 µL/s; n = 4 each), the 30G-pNFI-25/40 (n = 4), or the 30G-pNFI-35/40 (n = 4). 24 hours after administration, the testis, the epididymal head, and the epididymal tail were collected, and the Luc activity was measured. The results comparing the means + SD of the Luc activity in the testis, the epididymal head, and the epididymal tail are shown in FIGS. 12, 13 and 14, respectively.

As a result of administration using the 30G needle-equipped Hamilton syringe at an administration rate of 1 µL/s or 10 µL/s, the RFU values in the testis were 3.0 + 1.2 × 10⁵ and 8.2 ± 1.9 × 10⁵, respectively. When the 30G-pNFI-25/40 and the 30G-pNFI-35/40 were used, the RFU values in the testis were 6.1 ± 3.7 × 10⁷ and 7.3 ± 3.6 × 10⁷ respectively, showing similar levels.

### Test Example 9

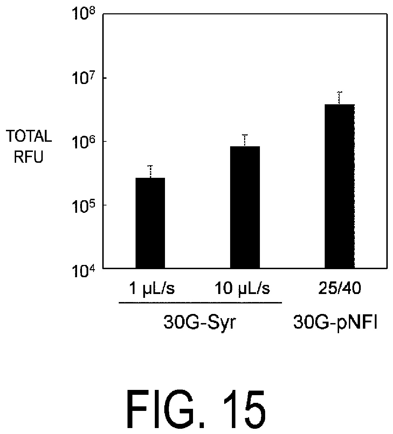

The pLuc with a concentration of 1 mg/mL was administered into the mouse liver left lateral lobe at a volume of 10 µL using a 30G needle-equipped Hamilton syringe (administration rate of 1 µL/s or 10 µL/s) or the 30G-pNFI-25/40. Each liver was collected 24 hours after administration, and the Luc activity was measured. The results comparing the means + SD (n = 4) are shown in FIG. 15.

The Luc activity increased in the following order: administration using the 30G needle-equipped Hamilton syringe at an administration rate of 1 µL/s, administration using the 30G needle-equipped Hamilton syringe at an administration rate of 10 µL/s, and administration using the 30G-pNFI-25/40. From these results, the Luc activity is considered to increase in an administration rate dependent manner.

## Claims

1. An injector for injecting a solution containing a biofunctional substance into an injection target, the injector comprising:
an accommodation part configured to accommodate the solution containing the biofunctional substance;
a pressurization part configured to pressurize the solution containing the biofunctional substance during actuation; and
an ejection part insertable into the injection target and including
an inlet port configured to facilitate inflow of the solution containing the biofunctional substance from the accommodation part when the solution containing the biofunctional substance is pressurized by the pressurization part, and
an outlet port configured to facilitate ejection of the flowing solution containing the biofunctional substance into the injection target,
wherein
an ejection rate of the solution containing the biofunctional substance exceeds 83.3 µL/s.

2. The injector according to claim 1, wherein the ejection rate of the solution containing the biofunctional substance is 200 µL/s or higher.

3. The injector according to claim 1 or 2, wherein an inner diameter of the ejection part is 0.41 mm or less.

4. The injector according to any one of claims 1 to 3, wherein an outer diameter of the ejection part is 0.72 mm or less.

5. The injector according to any one of claims 1 to 4, wherein the ejection part is an injection needle.

6. The injector according to any one of claims 1 to 5, wherein the biofunctional substance is one or more selected from the group consisting of a nucleic acid, a peptide, a protein, and a low-molecular-weight compound.

7. The injector according to any one of claims 1 to 6, wherein the injection target is one or more selected from the group consisting of an articular cavity, a spleen, an eyeball, skin, a muscle, a bone, cartilage, marrow, a ligament, a brain, a spinal cord, a lung, a liver, a heart, a kidney, a pancreas, a gall bladder, a digestive tract, a bladder, a reproductive organ, a lymph node, a lymphatic network, a blood vessel, a tumor arising in any one thereof, and mucosa and connective tissue associated with any one thereof.

8. A method comprising injecting a solution containing a biofunctional substance into an injection target, using the injector described in any one of claims 1 to 7.
